# EUROPEAN PATENT APPLICATION

(11) **EP 3 207 853 A1**
(43) Date of publication of application: **23.08.2017**
(21) Application number: 16807183.5
(22) Date of filing: 25.03.2016
(51) Int. Cl.: A61B 1/00

(54) **ENDOSCOPE**

(30) Priority: 08.06.2015 JP 2015116099
(71) Applicant: Olympus Corporation, Hachioji-shi Tokyo 192-8507 (JP)
(72) Inventor: FUJITANI Kiwamu, Hachioji-shi Tokyo 192-8507 (JP); HATANO Keisuke, Hachioji-shi Tokyo 192-8507 (JP); ITO Takayasu, Hachioji-shi Tokyo 192-8507 (JP); MATSUDA Eiji, Hachioji-shi Tokyo 192-8507 (JP)
(74) Representative: Schicker, Silvia
(86) International application number: PCT/JP2016/059701
(87) International publication number: WO 2016/199476

(57) **Abstract**

A dividing wall member 26 is provided inside a specific bending piece 25' selected from a plurality of bending pieces 25, an internal space of the bending piece 25' is divided by the dividing wall member 26 into a first space 27 that opens corresponding to one rotating shaft 25b, of rotating shafts 25b that form a left and right pair, and a second space 28 that is larger than the first space and opens corresponding to another rotating shaft 25b, of the rotating shafts 25b that form a left and right pair, and rotating shafts 25a that form an upper and lower pair, a signal cable 11a is inserted as a first internal component inside a first space 27, and a pair of light guides 12 and a treatment instrument insertion channel 13 are inserted as second internal components inside the second space 28.

## Description

### Technical Field

The present invention relates to an endoscope that includes a bending portion capable of bending toward a distal end side of an insertion portion.

### Background Art

Conventionally, endoscopes that can be inserted into a subject have been widely used in a medical field or an industrial field, for example, in order to observe a site inside an object which is difficult to observe, such as in-vivo of a body or an inside of a structure.

A bending portion for improving observability and insertability into a subject is provided on an insertion portion of such an endoscope. As the bending portion, a bending portion that is able to be bent and operated in a desired direction up, down, left, and right, by a bending knob or a bending lever or the like provided on an operation portion is widely employed.

In an endoscope provided with a bending portion that is able to bend in a desired direction up, down, left, and right in this way, in particular, it is highly likely that internal components will interfere with each other with the bending motion, so a countermeasure against damage to an internal component due to such interference or the like between internal components is desired. With respect to this, Japanese Patent Application Laid-Open Publication No. 2012-61221, for example, discloses technology in which a plurality of restricting members are provided on inner walls of cylindrical portions of a plurality of node rings (bending pieces) selected every predetermined number of node rings. The individual restricting members restrict movement in a radial direction of an internal component by protruding toward the insides of the cylindrical portions.

However, when a plurality of restricting members are provided inside the node rings, as in the technology described in Japanese Patent Application Laid-Open Publication No. 2012-61221 above, depending on an arrangement of the restricting members and the like, the restricting members may interfere with a jig or the like, and may therefore impair assemblability of an endoscope.

Also, even if movement in a radial direction of an internal component is restricted by the plurality of restricting members, as in the technology disclosed in Japanese Patent Application Laid-Open Publication No. 2012-61221 above, there are some cases in which it is difficult to sufficiently protect the respective internal components. For example, when a bending motion that rotates a distal end side is performed in a state in which a treatment instrument is inserted in a treatment instrument insertion channel, a portion of the treatment instrument insertion channel that has become more rigid due to the insertion of the treatment instrument may bulge out between restricting members, and as a result, another internal component such as an image pickup cable may press against an inner wall of a node ring or the like and be damaged.

In view of the foregoing situation, the present invention was made, and an object of the present invention is to provide an endoscope in which an internal component is able to be properly protected without impairing assemblability.

### Disclosure of Invention

### Means for Solving the Problem

An endoscope according to one aspect of the present invention includes a bending tube configured to be able to bend in any direction, in which a distal end side and a proximal end side of a plurality of bending pieces each having a ring shape are rotatably connected via opposing rotating shafts that form a front-rear respective pair and are disposed in an up-down direction or a left-right direction with respect to a central axis; a dividing wall member provided inside a specific bending piece selected from the plurality of bending pieces, the dividing wall member dividing an internal space of the specific bending piece into a first space that opens corresponding to one of the rotating shafts that form a front-rear respective pair, and a second space that is larger than the first space and opens corresponding to another of the rotating shafts; a first internal component configured to be inserted inside the first space; and a second internal component configured to be inserted inside the second space.

### Brief Description of the Drawings

Fig. 1 is a front view showing an external appearance of an endoscope;
Fig. 2 is a right side view showing an external appearance of the endoscope;
Fig. 3 is a top view showing an external appearance of the endoscope;
Fig. 4 is a sectional view showing main portions of a distal end portion and a bending portion;
Fig. 5 is a sectional view taken along line V-V in Fig. 4;
Fig. 6 is a sectional view taken along line VI-VI in Fig. 4;
Fig. 7 is a cross-sectional perspective view showing a bending piece broken longitudinally; and
Fig. 8 is a sectional view showing main portions of a flexible tube portion.

### Best Mode for Carrying Out the Invention

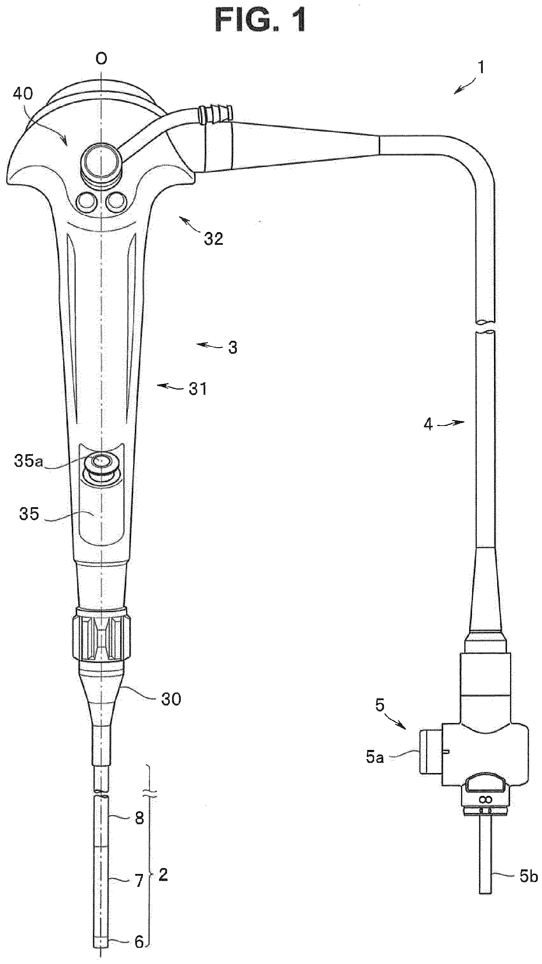

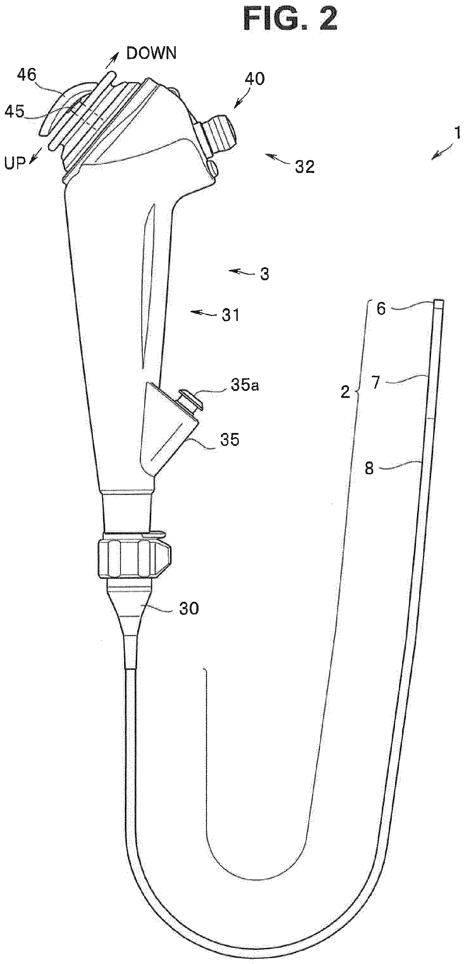

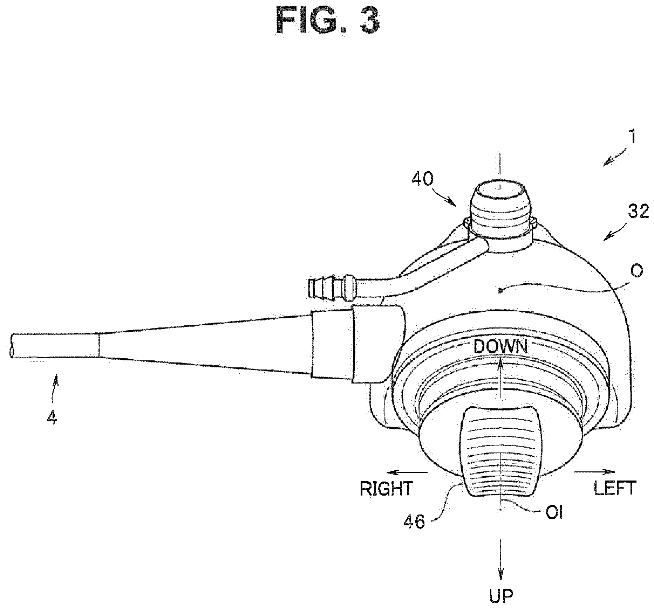

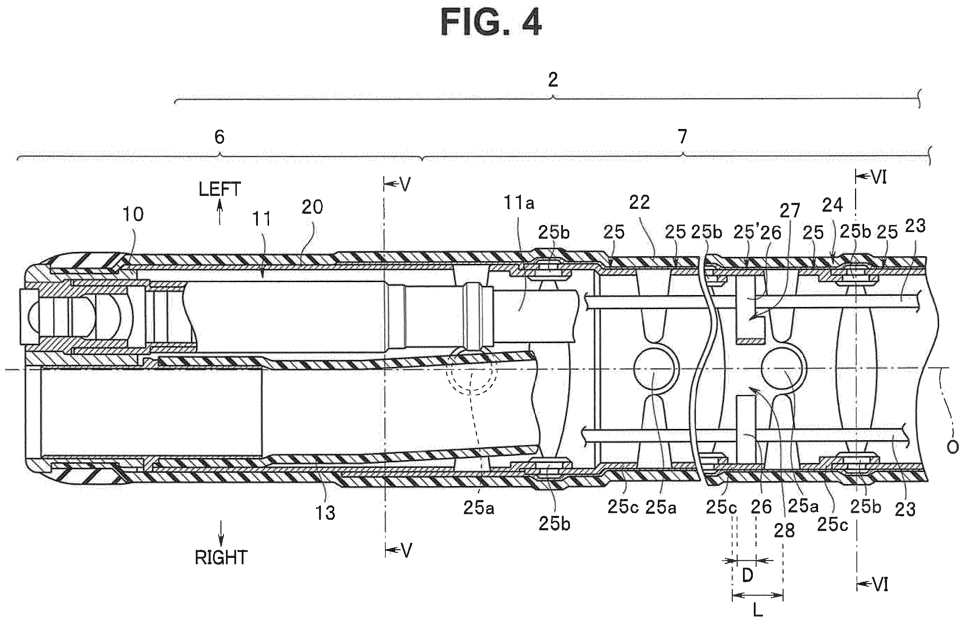

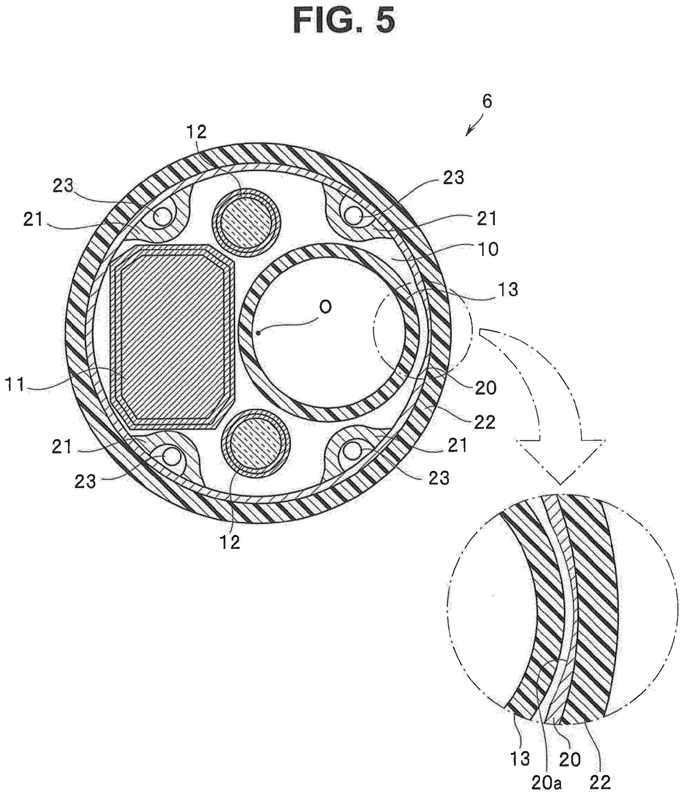

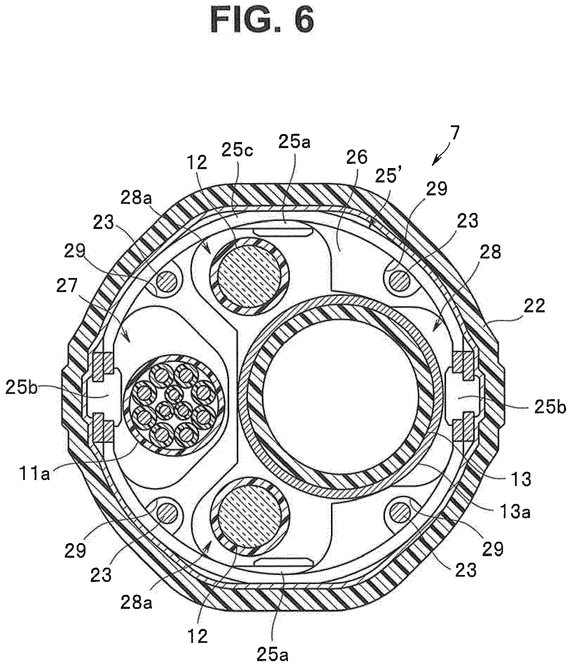

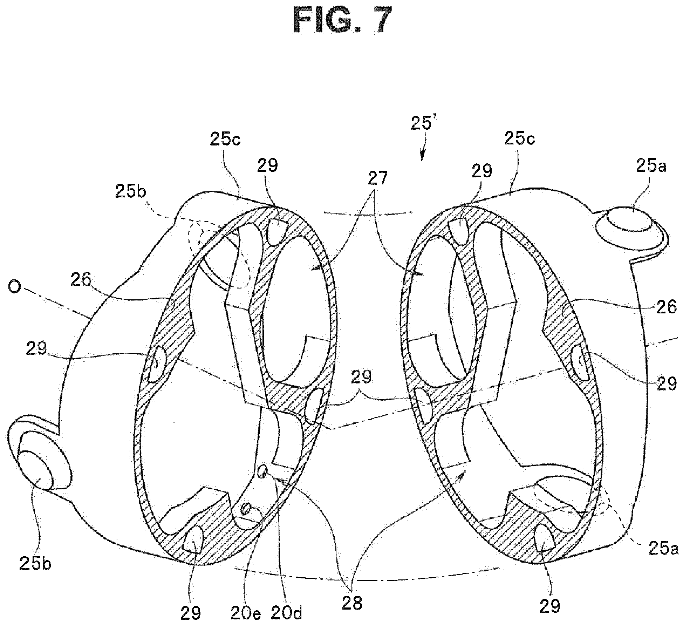

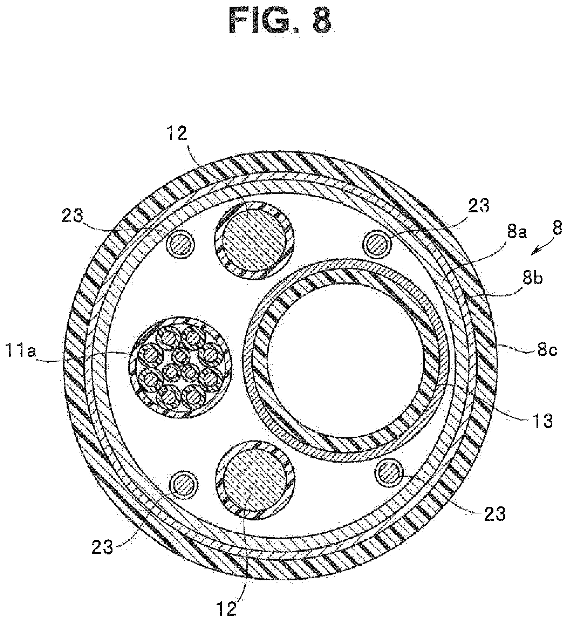

Hereinafter, an embodiment of the present invention will be described with reference to the drawings. The drawings relate to one embodiment of the present invention, with Fig. 1 being a front view showing an external appearance of an endoscope, Fig. 2 being a right side view showing an external appearance of the endoscope, Fig. 3 being a top view showing an external appearance of the endoscope, Fig. 4 being a sectional view showing main portions of a distal end portion and a bending portion, Fig. 5 being a sectional view taken along line V-V in Fig. 4, Fig. 6 being a sectional view taken along line VI-VI in Fig. 4, Fig. 7 being a cross-sectional perspective view showing a bending piece broken longitudinally, and Fig. 8 being a sectional view showing main portions of a flexible tube portion.

As shown in Figs. 1 and 2, an endoscope 1 of the embodiment is a bronchial endoscope. The endoscope 1 is provided with an insertion portion 2 formed in an elongated tube shape, an operation portion 3 continuously connected to a proximal end of the insertion portion 2, a universal cord 4 that is an endoscope cable extending from the operation portion 3, and an endoscope connector 5 arranged on a distal end of the universal cord 4.

The insertion portion 2 is configured by a tube-like member having flexibility, in which a distal end portion 6, a bending portion 7, and a flexible tube portion 8 are continuously connected in order from the distal end side.

For example, as shown in Figs. 4 and 5, a distal end rigid portion 10 made of metal is provided inside the distal end portion 6, and an image pickup unit 11 with a built-in image pickup device such as a CCD or CMOS, a plurality (for example, a pair) of light guides 12, and a treatment instrument insertion channel 13, are held by the distal end rigid portion 10.

Also, inside the distal end portion 6, a distal-most end bending piece 20 that has a generally cylindrical shape is externally fitted to a proximal end side of the distal end rigid portion 10, and an outer periphery of the distal-most end bending piece 20 is covered by a bending rubber 22. Wire fixing portions 21 are provided in four locations around an insertion axis (central axis) O, on an inner periphery of the distal-most end bending piece 20, and distal ends of four pull wires 23 inserted in the insertion portion 2 are fixed to the respective wire fixing portions 21, respectively.

Here, in order to efficiently dispose respective constituent members without increasing the diameter of the distal end portion 6, the image pickup unit 11 and the treatment instrument insertion channel 13, which are large members, are arranged lined up left and right inside the distal end rigid portion 10 and the distal-most end bending piece 20 (see Figs. 4 and 5), and the light guides 12 are disposed in spaces formed above and below, respectively, by the arrangement of the image pickup unit 11 and the treatment instrument insertion channel 13.

Also, in order to avoid interference between the image pickup unit 11 and the treatment instrument insertion channel 13, and the respective pull wires 23, the respective wire fixing portions 21 are provided in positions rotated and moved a predetermined angle around the insertion axis O with respect to up, down, left, and right positions of the distal end portion 6. That is, for example, the wire fixing portions 21 are provided in positions rotated and moved within a range of 15 to 75 degrees left and right, respectively, around the insertion axis O based on the up direction of the distal end portion 6, and positions rotated and moved within a range of 15 to 75 degrees left and right, respectively, around the insertion axis O based on the down direction of the distal end portion 6, on the distal-most end bending piece 20, as shown in Fig. 5.

The bending portion 7 is provided with a bending tube 24 configured to be able to actively bend in all directions around the insertion axis O, including the up, down, left, and right directions (UP-DOWN/RIGHT-LEFT), in accordance with operational input by an operator or the like with respect to the operation portion 3.

A signal cable 11a that extends from the image pickup unit 11, the pair of light guides 12, and the treatment instrument insertion channel 13 are inserted, together with the respective pull wires 23, in an arrangement that is substantially the same as the arrangement in the distal end portion 6, as internal components inside the bending tube 24. Furthermore, an outer periphery of the bending tube 24 is covered by the bending rubber 22 that extends from the distal end portion 6 side.

The flexible tube portion 8 includes a spiral sleeve 8a, a braid 8b that covers an outer periphery of the spiral sleeve 8a, and an outer skin 8c that covers the outer periphery of the braid 8b. The signal cable 11a and the treatment instrument insertion channel 13 described above are inserted inside the flexible tube portion 8. Furthermore, the pair of light guides 12 are inserted inside the flexible tube portion 8. Note that, although not shown, proximal end sides of the pair of light guides 12 are bundled into a single light guide on the proximal end side inside the flexible tube portion 8, or in the operation portion 3 or the like.

The operation portion 3 includes a bend preventing portion 30 connected to the flexible tube portion 8 in a state covering the proximal end of the flexible tube portion 8, and an operation portion main body 32 continuously connected to the bend preventing portion 30. Note that in the embodiment, directions around the insertion axis O of the operation portion 3 and the like are defined based on a state in which a user or the like is grasping a grasping portion 31. More specifically, with the operation portion 3, front, rear, left, and right directions (front surface, back surface, and left and right side surfaces, etc.) based on a user or the like grasping the grasping portion 31, are defined.

As shown in Fig. 1, a treatment instrument insertion portion 35 is provided on a front surface on a distal end side of the grasping portion 31. The treatment instrument insertion portion 35 has a treatment instrument insertion opening 35a into which various types of treatment instruments (not shown) are inserted. The treatment instrument insertion channel 13 is inserted, via a branch member, not shown, in the treatment instrument insertion opening 35a inside the operation portion 3. Also, a forceps plug (not shown), which is a lid member for closing the treatment instrument insertion opening 35a, is detachably attached to the treatment instrument insertion portion 35.

An operation button group 40 for executing various kinds of functions of the endoscope 1 is arranged on a front surface side of the operation portion main body 32. On the other hand, a bending lever 45 is arranged as a bending lever for performing a bending operation with respect to the bending portion 7, on a back surface side of the operation portion main body 32. Furthermore, the universal cord 4 is extending from one side portion (for example, a left side portion) of the operation portion main body 32.

Here, as shown in Figs. 2 and 3, the bending lever 45 of the embodiment is configured by a joystick-type lever that is able to tilt in all directions including up, down, left, and right directions, for example. A finger rest portion 46 that a thumb or the like of a user or the like can be made to abut against is provided on a protruding end portion of the bending lever 45. Also, when the bending lever 45 is tilt-operated through the finger rest portion 46, the respective pull wires 23 are suitably pulled or loosened by a wire pulling mechanism, not shown, arranged inside the operation portion main body 32, such that the bending portion 7 is able to be made to bend and move in a desired bending direction. Note that with the tilting directions of the bending lever 45, for example, a left-right direction of a tilting operation is defined as a left-right width direction of the operation portion 3, which is a direction orthogonal to the insertion axis O, and an up-down direction is defined as a direction orthogonal to the left-right width direction, as shown in Fig. 3, for example. Also, by performing a tilting operation in a circular motion with respect to the bending lever 45, a bending motion that rotates the distal end side of the bending portion 7 is also able to be realized.

The universal cord 4 is a composite cable through which various types of signal wires and the like that pass through the insertion portion 2 and reach the operation portion 3 from the distal end portion 6 side, and further extend from the operation portion 3, are inserted, and through which the light guides 12 of a light source device (not shown) are inserted.

The endoscope connector 5 includes an electrical connector portion 5a to which a signal cable that connects with a video processor (not shown) of an external device is connected, and a power supply connector portion 5b to which an electric cable and light guides that connect with the light source device that is an external device are connected.

Next, a detailed configuration of the bending portion 7 of the embodiment will be described.

As shown in Fig. 4, the bending tube 24 that configures the bending portion 7 includes a plurality of bending pieces 25 that are connected in series along the insertion axis O direction. That is, the respective bending pieces 25 each have a bending piece main body 25c that has a generally cylindrical shape (ring shape), and configure the bending tube 24 that is able to bend in any direction, by a distal end side and a proximal end side of the bending piece main body 25c being rotatably connected via opposing rotating shafts 25a or rotating shafts 25b that form a front-rear respective pair and are alternately differently disposed in an up-down direction or a left-right direction with respect to the central axis O.

In this kind of bending tube 24, a dividing wall member 26 is provided inside a specific bending piece 25 (hereinafter, this bending piece will be referred to as bending piece 25' to distinguish this bending piece from the others) selected from the plurality of bending pieces 25, as shown in Figs. 4, 6, and 7.

The dividing wall member 26 is configured by a plate-like member having a thickness D that is smaller than a length L in the insertion axis O direction of the bending piece main body 25c, for example, and is integrally formed inside the bending piece main body 25c (see Fig. 4). Also, two inner peripheral surfaces (wall surfaces) each having an annular shape are formed on the inside of the dividing wall member 26. An internal space of the bending piece main body 25c is divided into a first space 27 and a second space 28 by these wall surfaces.

Furthermore, wire guides 29 that pass through in the insertion axis O direction are drilled in positions near the first and second spaces 27 and 28 in the dividing wall member 26.

As shown in Fig. 6, the first space 27 is provided corresponding to one of the pair of rotating shafts 25b provided on the distal end side of the bending piece main body 25c, for example, and as a result, is provided in a position offset to a bending direction left side of the bending portion 7. Also, the signal cable 11 a that is a transmitting member for transmitting an image from the distal end side to the proximal end side of the insertion portion 2, for example, is inserted as a first internal component inside the first space 27.

Also, the second space 28 is formed corresponding to the other one of the pair of rotating shafts 25b provided on the distal end side of the bending piece main body 25c, and the pair of rotating shafts 25a provided on the proximal end side of the bending piece main body 25c, for example. As a result, the second space 28 is a space that is larger than the first space 27, and is formed in a position offset to a bending direction right side of the bending portion 7. Also, the pair of light guides 12 and the treatment instrument insertion channel 13, for example, are inserted as second internal components inside the second space 28.

Here, small spaces 28a that have a groove shape of a width sufficiently narrower than an outer diameter of the treatment instrument insertion channel 13 are integrally formed in positions offset to the bending direction upper side and lower side, respectively, of the bending portion 7, for example, in the second space 28. Also, the pair of light guides 12 are disposed inside these small spaces 28a, respectively.

Also, in order to prevent damage to the respective internal components due to interference with the dividing wall member 26, portions against which the respective internal components (the signal cable 11a, the light guides 12, and the treatment instrument insertion channel 13, etc.) abut, of the wall surface formed by the dividing wall member 26, are preferably all set to obtuse angles. Moreover, a protective member such as a coil 13a having a predetermined pitch is preferably wound around an outer periphery of the treatment instrument insertion channel 13. The pitch of the coil 13a or the like is preferably set smaller than the thickness D of the dividing wall member 26. By setting the pitch of the coil 13a or the like in this way, the dividing wall member 26 reliably abuts against the coil 13a or the like, and does not directly contact the treatment instrument insertion channel 13, so durability of the treatment instrument insertion channel 13 is improved. Note that in order to prevent incorrect assembly when assembling the bending tube 24, an indicator (for example, an UP indicator) that indicates a predetermined direction is preferably provided on the bending piece 25'. As such an indicator, a set of a through-hole 20d and a closed end hole 20e may be used, as shown in Fig. 7, for example, and misidentification of the up, down, left, and right directions when assembling the bending tube 24 is able to be prevented with a simple configuration, by disposing the set of the through-hole 20d and the closed end hole 20e lined up in a predetermined direction.

Also, the connecting portion of the image pickup unit 11 and the signal cable 11a typically tends to increase in diameter. In order to prevent interference between the rotating shafts and the portion that is increased in diameter in this way, the pair of rotating shafts positioned on the distal-most end are preferably disposed in positions away from the image pickup unit 11. Therefore, in the embodiment in which the first space 27 is set corresponding to one of the left and right pair of rotating shafts 25b, as shown in Fig. 4, for example, the upper and lower pair of rotating shafts 25a are employed as rotating shafts that connect the distal-most end bending piece 20. Also, in particular, in order to efficiently realize a reduction in diameter of the insertion portion 2 while preventing interference between the treatment instrument insertion channel 13 and the distal-most end bending piece 20 in the distal-most end bending piece 20 and the like in which the image pickup unit 11 and the treatment instrument insertion channel 11 are provided together, a concave portion 20a for preventing interference with the treatment instrument insertion channel 13 is preferably provided on an inner wall surface of the distal-most end bending piece 20, as shown in Fig. 5, for example.

According to this kind of embodiment, an internal component is able to be properly protected without impairing assemblability, by providing the dividing wall member 26 inside the specific bending piece 25' selected from the plurality of bending pieces 25, dividing the internal space of the bending piece 25' by the dividing wall member 26 into the first space 27 that opens corresponding to one rotating shaft 25b, of the rotating shafts 25b that form a left and right pair, and the second space 28 that is larger than the first space and opens corresponding to the other rotating shaft 25b, of the rotating shafts 25b that form a left and right pair, and the rotating shafts 25a that form an upper and lower pair, inserting the signal cable 11a as the first internal component inside the first space 27, and inserting the pair of light guides 12 and the treatment instrument insertion channel 13 as the second internal components inside the second space 28.

That is, by inserting the signal cable 11 a that is most likely to be damaged or the like, of the internal components inserted in the bending portion 7, inside the first space 27, and inserting the treatment instrument insertion channel 13 that is the largest and most likely to affect (interfere with) another internal component, of the internal components inserted in the bending portion 7, inside the second space 28, the respective internal components are able to be properly protected, even in the endoscope 1 in which a bending motion that rotates the distal end side of the bending portion 7 is possible, in particular. That is, by dividing up and inserting the signal cable 11a and the treatment instrument insertion channel 13 into the first space 27 and the second space 28 that are independent of each another, even when the bending portion 7 is made to rotate in a state in which a treatment instrument is inserted in the treatment instrument insertion channel 13, or the like, interference between the signal cable 11 a and the treatment instrument insertion channel 13 that has become more rigid is able to be properly avoided.

In this case, the first and second spaces 27 and 28 are open in positions corresponding to the respective rotating shafts 25a and 25b, so even when connecting the respective bending pieces 25 by the respective rotating shafts 25a and 26b, the dividing wall member 26 is able to be prevented from inhibiting insertion of a jig or the like, so good assemblability is able to be obtained.

Also, even when the pair of light guides 12, the proximal end sides of which are bundled into one, are inserted into the bending tube 24 from the distal end side, by inserting these light guides 12 all together in the second space 28 that is a large space, good operability is able to be maintained even when the insides of the bending pieces 25 are divided. Moreover, by integrally forming the small spaces 28a for which the width is set smaller than the outer diameter of the treatment instrument insertion channel 13 inside the second space 28, and moving and housing the light guides 12 after insertion into the second space 28 in the small spaces 28a, these light guides 12 are also able to be well protected from the treatment instrument insertion channel 13.

Note that the present invention is not limited to the respective embodiments described above. Various changes and modifications are possible, and these are also within the technical scope of the present invention. For example, in the embodiment described above, a bronchial endoscope is given as an example, but the present invention is not limited to this, and may of course also be applied to another endoscope.

Also, in the embodiment described above, the signal cable 11 a for transmitting an image pickup signal acquired by the image pickup unit 11 is given as an example of a transmitting member (internal component) for transmitting an image, but the present invention is not limited to this. For example, an image guide may also be employed.

The present application claims the priority of Japanese Patent Application No. 2015-116099, filed in Japan on June 8, 2015, the disclosure of which is incorporated by reference herein in its entirety.

## Claims

1. An endoscope comprising:
a bending tube configured to be able to bend in any direction, in which a distal end side and a proximal end side of a plurality of bending pieces each having a ring shape are rotatably connected via opposing rotating shafts that form a front-rear respective pair and are disposed in an up-down direction or a left-right direction with respect to a central axis;
a dividing wall member provided inside a specific bending piece selected from the plurality of bending pieces, the dividing wall member dividing an internal space of the specific bending piece into a first space that opens corresponding to one of the rotating shafts that form a front-rear respective pair, and a second space that is larger than the first space and opens corresponding to another of the rotating shafts;
a first internal component configured to be inserted inside the first space; and
a second internal component configured to be inserted inside the second space.

2. The endoscope according to claim 1, wherein:
the first internal component includes a transmitting member configured to transmit an image; and
the second internal component includes a treatment instrument insertion channel configured such that a treatment instrument is inserted through the treatment instrument insertion channel.

3. The endoscope according to claim 2, wherein the second internal component further includes a plurality of light guides, proximal end sides of which are bundled into one.

4. The endoscope according to claim 3, wherein:
the second space integrally includes a plurality of small spaces configured such that the plurality of light guides are inserted through the plurality of small spaces, respectively; and
a width of the small spaces is set smaller than an outer diameter of the treatment instrument insertion channel.

5. The endoscope according to claim 1, wherein a portion against which the first internal component and the second internal component abut, of a wall surface formed by the dividing wall member, is formed at an obtuse angle.

6. The endoscope according to claim 1, wherein the pair of rotating shafts positioned on a distal-most end of the bending tube is a pair of rotating shafts disposed in a direction different from a direction of the rotating shafts corresponding to the first space.
